# EUROPÄISCHE PATENTSCHRIFT

(11) **EP 0 948 475 B1**
(45) Veröffentlichungstag und Bekanntmachung des Hinweises auf die Patenterteilung: **26.03.2003**
(21) Anmeldenummer: 97953761.0
(22) Anmeldetag: 09.12.1997
(51) Int. Cl.: C07C 5/333, B01J 21/06, B01J 23/40, B01J 23/36, B01J 23/24

(54) **VERFAHREN ZUR HERSTELLUNG VON OLEFINEN, INSBESONDERE VON PROPYLEN, DURCH DEHYDRIERUNG**
METHOD FOR PRODUCING OLEFINS, IN PARTICULAR PROPYLENES, BY DEHYDROGENATION
PROCEDE DE PRODUCTION D'OLEFINES, EN PARTICULIER DE PROPYLENE, PAR DESHYDROGENATION

(30) Priorität: 27.12.1996 DE 19654391
(43) Veröffentlichungstag der Anmeldung: 13.10.1999
(73) Patentinhaber: BASF AKTIENGESELLSCHAFT, 67056 Ludwigshafen (DE)
(72) Erfinder: HEINEKE, Daniel, D-67065 Ludwigshafen (DE); BAIER, Michael, D-68161 Mannheim (DE); DEMUTH, Dirk, D-68161 Mannheim (DE); HARTH, Klaus, D-67317 Altleiningen (DE)
(86) Internationale Anmeldenummer: EP9706858
(87) Internationale Veröffentlichungsnummer: WO98029365

(56) Entgegenhaltungen:
- EP-A- 0 441 430
- EP-A- 0 559 509
- EP-A- 0 730 906
- WO-A-89/04717
- WO-A-90/06907
- BE-A- 421 888
- BE-A- 809 873
- DE-A- 19 646 538
- US-A- 2 098 959
- US-A- 2 375 021

## Beschreibung

Propylen wird gegenwärtig im wesentlichen aus dem beim Steamcracking von leichtem Naphtha anfallenden Produktgemisch gewonnen. Wirtschaftliche und andere Gründe machen eine Flexibilisierung der Rohstoffbasis wünschenswert. Eine Alternative zur Gewinnung von Propylen ist die Dehydrierung von Propan.

Auf nichtoxidativem Weg kann Propylen durch Dehydrierung von Propan an Edelmetall-Katalysatoren wie Pt/Al₂O_{3,} Pt/Sn/Al₂O₃ oder an edelmetallfreien Katalysatoren wie Cr/Al₂O₃ erhalten werden. Die Umsetzung ist stark endotherm und läuft nur bei hoher Temperatur mit befriedigender Geschwindigkeit ab. Dabei werden Nebenreaktionen begünstigt, z.B. Abbau des Propans zu Ethylen und Methan; gleichzeitig wird Ethylen durch den bei der Dehydrierung freigesetzten Wasserstoff hydriert. Die Selektivität der Umsetzung nimmt wegen der nebenproduktabhängigen Konkurrenzreaktionen mit steigendem Umsatz stark ab, was die technische Durchführbarkeit des Verfahrens infragestellt. Nebenreaktionen führen außerdem zur Verkokung der verwendeten Katalysatoren, die nach relativ kurzen Betriebszeiten regeneriert werden müßten.

Zur technischen Reife ist ein Verfahren gelangt, in dem bei niedrigem Druck und relativ hoher Temperatur gearbeitet und der Katalysator kontinuierlich mit Luftsauerstoff regeneriert wird (Energy Prog. (1986), 6(3) 171-6 und Chem. Eng. Today, Copying Uncertainty, Aust. Chem. Eng. Conf. 11th (1983), 663- 71). Das Verfahren kann mit Pt/Al₂O₃-Katalysatoren in einem Wanderbett bei 600-700°C und einem Druck von 2-5 bar ausgeübt werden.

Bei dem in WO 9523123 beschriebenen Verfahren werden Cr/Al₂O₃-Katalysatoren verwendet, die cyclisch, d.h. im Regenerativ-Verfahren betrieben werden. Dabei wird mit der Abwärme, die beim Abbrennen des Kohlenstoffs frei wird, das Propan vorgeheizt. Pt/Sn/Al₂O₃-Katalysatoren sind bekannt aus Shiyou Huagong (1992), 21(8), 511-515. Dort ist auch beschrieben, daß diese Katalysatoren mit Kalium oder Magnesium dotiert werden können. Durch Dotierung mit Zinn soll die Desaktivierung trotz Verkokung verlangsamt werden (Stud. Surf. Sci. Catal. 1994, 88, 519-24).

Aus der BE-A-421 888 ist ein Verfahren zur Herstellung von Olefin durch Dehydrierung von entsprechenden Alkanen in Gegenwart von Katalysatoren, die Oxide von Titan, Zirkon oder Hafnium enthalten, bekannt.

Aus der EP-A-441 430 ist ein Katalysator für die Dehydrierung von Propan bekannt, der aus Platin und gegebenenfalls Zinn auf einem Titan enthaltenden Träger besteht.

Aus der WO-A-90/06907 ist ein Verfahren zur Dehydrierung von Kohlenwasserstoffen in Gegenwart von einer Katalysatorzusammensetzung bekannt, die Chromoxid, Aluminiumoxid, Alkali- und/oder Erdalkalimetallverbindungen und eine Metallverbindung der dritten und/oder vierten Nebengruppe des PSE umfaßt.

Oxidische Katalysatoren mit redoxaktiven Elementen, die nicht in ihrer niedrigsten Oxidationsstufe vorliegen, beschreibt EP-A-403 462.

Die Dehydrierung von Propan mit Zeolithen vom ZSM-5-Typ ist ebenfalls bekannt. Werden diese Zeolithe mit Zink dotiert, so hat dies Einfluß auf das Säure-Basen-Verhalten der Zeolithe: Crackreaktionen sollen weitgehend unterdrückt werden (J. Chin. Inst. Chem. Eng. (1990), 21(3), 167-72).

Die bekanntgewordenen Verfahren haben vor allem den Nachteil, daß mit steigendem Umsatz die Selektivität stark nachläßt. Außerdem müssen die Katalysatoren häufig regeneriert werden, was äußerst nachteilig für ein technisches Verfahren ist.

Die Erfindung hat zur Aufgabe, den vorgenannten Nachteilen bekannter Verfahren abzuhelfen und Katalysatoren bereitzustellen, die ein Verfahren zur Herstellung insbesondere von Propylen und anderen niedermolekularen Olefinen durch Dehydrierung entsprechender Paraffinkohlenwasserstoffe ermöglichen und auch bei hohem Umsatz hohe Selektivität erzielen.

Die Aufgabe wurde gelöst durch einen Katalysator, enthaltend
a) mindestens ein Oxid mindestens eines Elements aus der Gruppe IV B des Periodensystems der Elemente mit mehr als 90 % einer kristallinen Modifikation,
b) mindestens ein Element aus den Gruppen VIII und VIB,
c) Rhenium und/oder Zinn und
d) mindestens eine Verbindung eines Elements aus den Gruppen I A, II A, III A, III B und/oder von Zink,
und ein Verfahren zur Herstellung von Olefinen mit zwei bis fünf Kohlenstoffatomen in der längsten Kette durch Dehydrierung entsprechender Paraffinkohlenwasserstoffe an einem Katalysator, das dadurch gekennzeichnet ist, daß der Katalysator
a) mindestens ein Oxid mindestens eines Elements aus der Gruppe IV B des Periodensystems der Elemente mit mehr als 90 % einer kristallinen Modifikation,
b) mindestens ein Element aus den Gruppen VIII und VI B,
c) Rhenium und/oder Zinn und
d) mindestens eine Verbindung eines Elements aus den Gruppen I A, II A, III A, III B und/oder von Zink
enthält.

Als Oxid mindestens eines Elements aus der Gruppe IV B eignen sich insbesondere Zirkonoxid (ZrO₂) und Titanoxid (TiO₂). Das Oxid kann mit 0,005 bis 5 Gew.-% Palladium, Platin, und/oder Rhodium dotiert sein. Als dehydrieraktive Elemente sind vor allem Metalle der Gruppe VIII geeignet, wobei sich insbesondere die Edelmetalle Platin und Palladium eignen, bevorzugt Platin.

Wenn ein Edelmetall als dehydrieraktives Element verwendet wird, können zusätzlich Metalle eingesetzt werden, die das Sintern des Edelmetalls verlangsamen können, wie Rhenium, Ir und Sn, insbesondere Re und Sn.

Als weitere Elemente kommen solche infrage, von denen bekannt ist, daß sie die Azidität der Katalysatoroberfläche beeinflussen oder Edelmetalle gegen Sintern stabilisieren können. Solche weiteren Elemente sind alle Elemente der Gruppen I A und II A, d.h. Li, Na, K, Rb, Cs einerseits und Mg, Ca, Sr und Ba andererseits. Als Elemente der Gruppe III A kommen insbesondere Gallium, Indium oder Thallium in Frage. Als Elemente der Gruppe III B kommen insbesondere Y und La sowie Seltenerd-Elemente infrage. Als wirksam hat sich auch Zink erwiesen.

Die Verwendung mindestens eines Oxids mindestens eines Elements aus der Gruppe IV B ist für die Zweck der Erfindung wesentlich, während die anderen Bestandteile lediglich für die Grundreaktion von Bedeutung sind und unterstützend wirken. So können anstelle eines Edelmetalls auch andere dehydrieraktive Metalle, zum Beispiel der Gruppe VI B, insbesondere Chrom oder Molybdän zugegen sein.

Wesentlich für die Erfindung ist, daß die kristalline Phase des Zirkonoxids unter den Bedingungen der Dehydrierung stabil ist. Geht man von tetragonalem ZrO₂ aus, so kann dies durch die Dotierung mit La oder Y unterstützt werden.

Die Porenweite der Katalysatoren beträgt 2 bis 60 nm, wobei mindestens 10 % der Poren eine Weite von mehr als 20 nm aufweisen und das spezifische Porenvolumen 0,1 bis 1 ml/g beträgt.

Gegenüber den bekannten Katalysatoren weisen die erfindungsgemäßen Katalysatoren bei der Dehydrierung von Propan zu Propylen den Vorteil höherer Selektivität bei gleichzeitig höherem Umsatz auf. Außerdem erweist sich als Vorteil, daß die erfindungsgemäßen Katalysatoren ohne zusätzlichen Wasserstoff betrieben werden können, der sonst zur Unterdrückung der Verkokung eingesetzt werden müßte. Weitere Vorteile sind ihre hohe mechanische Festigkeit, hohe Standzeit und leichte Formgebung.

Zur Herstellung der erfindungsgemäßen Katalysatoren können amphotere Oxide des Zirkons und des Titans oder deren Mischungen oder geeignete Vorprodukte (Precursoren) eingesetzt werden, die sich durch Calcinieren in die Oxide umwandeln lassen.

Das Herstellverfahren kann nach bekannten Vorbildern gewählt werden, zum Beispiel nach dem Sol-Gel-Verfahren, Fällung der Salze, Entwässern der entsprechenden Säuren, Trockenmischen, Aufschlämmen oder Sprühtrocknen.

Die Dotierung mit einer basischen Verbindung kann entweder während der Herstellung, zum Beispiel durch gemeinsames Fällen oder nachträglich durch Tränken des keramischen amphoteren Oxids mit einer Verbindung der betreffenden Alkali- oder Erdalkalimetallverbindung etc. erfolgen.

Der dehydrieraktive Bestandteil wird in der Regel durch Tränkung mit einer geeigneten Verbindung des betreffenden Elements aufgebracht. Eine solche Verbindung wird so gewählt, daß sie sich durch Calcinieren in das entsprechende Metalloxid umwandeln läßt. Statt durch Tränkung kann die dehydrieraktive Komponente aber auch durch andere Verfahren wie beispielsweise Aufsprühen erfolgen. Geeignete Metallsalze sind z.B. die Nitrate, Acetate und Chloride der entsprechenden Metalle, möglich sind auch komplexe Anionen der verwendeten Metalle. Bevorzugt werden H₂PtCl₆ oder Pt(NO₃)₂ für Platin und Cr(NO₃)₃ oder (NH₄)₂CrO₄ für Chrom eingesetzt. Geeignete Precursoren im Falle der Verwendung von Edelmetallen als dehydrieraktive Komponente sind auch die entsprechenden Edelmetallsole, die nach einem der bekannten Verfahren, zum Beispiel durch Reduktion eines Metallsalzes in Gegenwart eines Stabilisators wie PVP mit einem Reduktionsmittel hergestellt werden können. Die Herstellung wird in DE 195 00 366 ausführlich behandelt.

Der Katalysator kann fest angeordnet oder z.B. in Form eines Wirbelbetts verwendet werden und eine entsprechende Gestalt haben. Geeignet sind z.B. Formen wie Splitt, Tabletten, Monolithe, Kugeln oder Extrudate (Stränge mit entsprechendem Querschnitt wie Wagenrad, Stern, Ring).

Der Gehalt an Alkali-, Erdalkalimetall oder an einem Metall der Gruppen III A oder III B einschließlich der seltenen Erden oder Zink liegt bei bis zu 20 Gew.-%, bevorzugt zwischen 1 und 15 Gew.-%, besonders bevorzugt zwischen 1 und 10 Gew.-%. Als Alkaliund Erdalkalimetallprecursor verwendet man zweckmäßig Verbindungen, die sich durch Calcinieren direkt in die entsprechenden Oxide umwandeln lassen. Geeignet sind zum Beispiel Hydroxid, Carbonat, Oxalat, Acetat oder gemischte Hydroxycarbonate.

Wird der keramische Träger zusätzlich oder ausschließlich mit einem Metall der gruppen III A oder III B dotiert, so sollte man auch in diesem Fall von Verbindungen ausgehen, die sich durch Calcinieren in die entsprechenden Oxide umwandeln lassen. Wird Lanthan verwendet, so sind beispielsweise Lanthan-Oxid-Carbonat, La(OH)₃ La₃(CO₃)₂, La(NO₃)₃ oder Lanthanverbindungen die organische Anionen enthalten, wie La-Acetat, La-Formiat oder La-Oxalat geeignet.

Der Gehalt der Katalysatoren an einer dehydrieraktiven Komponente beträgt bis zu 10 Gew.-%. Wird der Katalysator mit einem dehydrieraktiven Element der Gruppe VIII als dehydrieraktivem Element dotiert, so beträgt der Gehalt bevorzugt 0.2 bis 8 Gew.-%, besonders bevorzugt 0.5 bis 2 Gew.-%. Wird der Katalysator mit einem Edelmetall als dehydrieraktive Komponente dotiert, so beträgt der Gehalt bevorzugt 0.2 bis 2 Gew.-%, besonders bevorzugt 0.5 bis 1.5 Gew.-%.

Die Katalysatoren weisen eine BET-Oberfläche von 10 bis zu 500 m²/g oder mehr, bevorzugt von 10-300 m²/g, besonders bevorzugt von 20-100 m²/g auf. Das Porenvolumen liegt in Regel zwischen 0.1 und 1 ml/g, bevorzugt von 0.15 bis 0.6 ml/g, besonders bevorzugt von 0.2 bis 0.4 ml/g. Der mittlere, durch Hg-Penetrationsanalyse bestimmbare Porendurchmesser liegt zwischen 0.008 und 0.06 µm, bevorzugt zwischen 0.01 und 0.04 µm.

Die Propan-Dehydrierung wird bei Temperaturen von 300-800°C, bevorzugt 450-700°C,und bei Drücken von 10 mbar bis 100 bar, bevorzugt 100 mbar bis 40 bar mit einer WHSV (Weight Hourly Space Velocity; in [(g Edukt)·(g Kat)⁻¹·h⁻¹]) von 0.01 bis 100, bevorzugt 0.1 bis 20 durchgeführt. Neben dem zu dehydrierenden Kohlenwasserstoff können Verdünnungsmittel wie beispielsweise CO₂, N₂, Edelgase oder Dampf zugegen sein. Gegebenenfalls, d.h bei scharfen Reaktionsbedingungen kann zum Kohlenwasserstoffstrom Wasserstoff zugegeben werden, wobei das Verhältnis von Wasserstoff zu Kohlenwasserstoffstrom von 0.1 bis 100 bevorzugt von 1-20 betragen kann. Der zugesetzte Wasserstoff dient dazu, den auf der Oberfläche des Katalysators durch Verkokung entstehenden Kohlenstoff zu entfernen.

Neben der kontinuierlichen Zugabe eines Gases, welches die Verkokung während der Reaktion verhindert, gibt es die Möglichkeit, den Katalysator durch Überleiten von Wasserstoff oder Luft von Zeit zu Zeit zu regenerieren. Die Regenerierung selbst findet bei Temperaturen im Bereich 300-900°C, bevorzugt 400-800°C mit einem freien Oxidationsmittel, vorzugsweise mit Luft oder in reduktiver Atmosphäre vorzugsweise mit Wasserstoff statt. Die Regenerierung kann bei Unterdruck, atmosphärischem Druck oder Überdruck betrieben werden. Bevorzugt sind Drucke im Bereich 500 mbar bis 100 bar.

### Beispiele:

### Katalysatorherstellung:

### Vergleichsbeispiel 1-4

Zu einer Lösung von 24.85 g ZrOCl₂·H₂O und 1.33 g La(NO₃)₃·6H₂O in 50 ml Wasser wurde unter Rühren eine 4 M NH₃-Lösung gegeben, bis keine Niederschlagsbildung mehr zu beobachten war. Der Niederschlag wurde abfiltriert, mit Wasser chloridfrei gewaschen und 16 Stunden bei 120°C getrocknet. Der getrocknete Niederschlag wurde in 50 ml einer 0.02 M (NH₄)₂CrO₄-Lösung suspendiert und die überstehende Lösung bei 50°C eingedampft. Der Rückstand wurde 16 Stunden bei 120°C getrocknet und 4 Stunden bei 600°C calciniert. Der fertige Katalysator enthielt 0.66% Chrom und 5.3% Lanthan. Die kristalline Phase der Zirkondioxide wurde röntgenographisch als überwiegend tetragonal bestimmt. Die Primärteilchengröße des Zirkondioxids wurde mit TEM zu etwa 5 nm bestimmt.

In Beispiel 1 wurde der frische Katalysator verwendet. Für Beispiel 2 wurde der gleiche Katalysator nach Regenerierung bei 500°C mit Luftsauerstoff verwendet. Für Beispiel 3 wurde der zum zweiten Mal, für Beispiel 4 zum dritten Mal mit Luftsauerstoff regenerierte Katalysator eingesetzt.

### Vergleichsbeispiel 5 und 6

Ein Katalysator wurde durch Tränkung von ZrO₂ (Träger SN 9316335, Fa. Norton, 46 m²/g, weitgehend monoklin) mit Pt(NO₃)₂ und Sn(OAc)₂ hergestellt. Der Pt-Gehalt betrug 1 Gew.-%, der Sn-Gehalt 0.5 Gew.-%. Der Katalysator wurde 3 Stunden bei 650°C calciniert.

### Vergleichsbeispiel 7

Ein Katalysator wurde durch Tränkung eines weitgehend monoklinen ZrO₂ (Träger SN 9316321, Fa. Norton, 49 m²/g) mit einer Lösung von 0.821 g Cr(NO₃)₃ x 9H₂O in 2.5 ml Wasser und anschließendes Tränken mit einer Lösung von 1.763 g La(NO₃)₃ in 2.5 ml Wasser hergestellt. Der Katalysator wurde 16 Stunden bei 120°C getrocknet und 2 Stunden bei 500°C calciniert. Der fertige Katalysator wies einen Chrom-Gehalt von 0.9% und einen Lanthan-Gehalt von 4.5 Gew.-% auf.

### Vergleichsversuche V1 - V4

Die Vergleichskatalysatoren (V1: 10% Cr/Al₂O₃, V2: 1% Cr/Al₂O₃ und V3: 5% Cr/Al₂O₃) wurden durch Tränkung von α-Al₂O₃ (9.5 m²/g) mit unterschiedlichen Mengen Cr(NO₃)₃ hergestellt. Diese Katalysatoren wurden 6 Stunden bei 120°C getrocknet und anschließend 2 Stunden bei 500°C calciniert. Der Vergleichskatalysator V4 wurde durch Tränkung des gleichen Al₂O₃-Trägers mit Pt(NO₃)₂ hergestellt. Der Katalysator wurde 16 Stunden bei 120°C getrocknet und anschließend 2 Stunden bei 500°C calciniert.

### Dehydrierung

Die Dehydrierung wurde in einem Mikrofestbett-Pulsreaktor bei einer Temperatur von 500°C durchgeführt. Dabei wurden etwa 0.6 g des Katalysators in ein Mikrofestbett eingewogen und pulsierend d.h. mit einem regelmäßig unterbrochenen Strom von einem Propangas ohne Zusatz von Wasserstoff (ohne H₂) bei atmosphärischem Druck beaufschlagt. Die Reaktionsprodukte wurden für jeden Puls mittels on-line GC quantitativ erfaßt. Zwischen zwei aufeinanderfolgenden Propan-Pulsen (ca. 1,5 min Abstand) strömte Helium-Trägergas durch den Reaktor.

Ein einzelner Puls enthielt ca. 100 µl Propan. Die Strömungsgeschwindigkeit des Trägergases betrug ca. 21,5 ml/min. Die Verweilzeit betrug je nach Schütthöhe des Katalysators (10 bis 25 mm) etwa 1 bis 2 Sekunden. Die Belastung des Katalysators (WHSV; siehe oben) während eines Pulses betrug, ebenfalls in Abhängigkeit von der Schütthöhe, 1.7 bis 3.4. Die erzielten Ergebnisse können Tabelle 1 entnommen werden und beziehen sich auf den maximal erreichten Umsatz.

**Tab. 1:**

| Katalysatorleistung bei Propan-Dehydrierung im Pulsreaktor | | | | | | |
|---|---|---|---|---|---|---|
| Beispiel | Katalysator | Verweilzeit [s] | Schütthöhe | A[%] | U [%] | Sel. [%] |
| 1 | La/Cr/ZrO₂ | 0.8 | 15 mm | 50 | 54 | 92 |
| 2 | La/Cr/ZrO₂ | 0.8 | 15 mm | 49 | 53 | 92 |
| 3 | La/Cr/ZrO₂ | 0.8 | 15 mm | 47 | 51 | 92 |
| 4 | La/Cr/ZrO₂ | 0.8 | 15 mm | 49 | 53 | 92 |
| 5 | 1%Pt/0.5%Sn/ZrO₂ | 1.2 | 23 mm | 49 | 53 | 92 |
| 6 | 1% Pt/0.5% Sn/ ZrO₂ | 1.2 | 23 mm | 43 | 44 | 97 |
| 7 | La/Cr/ZrO₂ | 1.3 | 24 mm | 35 | 36 | 96 |
| V 1 | 10 % Cr/Al₂O₃ | 1.3 | 25 mm | 25 | 26 | 96 |
| V 2 | 1% Cr/ Al₂O₃ | 1.3 | 24 mm | 14 | 18 | 79 |
| V 3 | 5 % Cr/ Al₂O₃ | 1.2 | 23 mm | 22 | 23 | 95 |
| V 4 | 1 % Pt/ Al₂O₃ | 1.3 | 24 mm | 5 | 59 | 9 |

Es ist darauf hinzuweisen, daß der gegenüber der Gleichgewichtslage (500°C) wesentlich höhere Umsatz durch den Pulsbetrieb Pulsfahrweise erzielt wird, bei dem sich aufgrund der kurzen Verweilzeit und dem Abstand von etwa 1.5 min zwischen den Pulsen kein thermodynamisches Gleichgewicht einstellt. Dennoch gestattet diese Methode eine gute Vergleichbarkeit der Selektivität bei hohem Umsatz.

Mit den erfindungsgemäßen Katalysatoren werden bei gleicher Temperatur höhere Umsätze als bei den Vergleichskatalysatoren bei vergleichbar hoher Selektivität erzielt. Die Ausbeuten liegen deshalb bei den erfindungsgemäßen Katalysatoren signifikant höher als bei den Vergleichskatalysatoren.

## Patentansprüche

1. Verfahren zur Herstellung von Olefinen mit zwei bis fünf Kohlenstoffatomen in der längsten Kette durch Dehydrierung entsprechender Paraffinkohlenwasserstoffe an einem Katalysator, **dadurch gekennzeichnet, daß** der Katalysator
a) mindestens ein Oxid mindestens eines Elements aus der Gruppe IV B des Periodensystems der Elemente mit mehr als 90 % einer kristallinen Modifikation,
b) mindestens ein Element aus den Gruppen VIII und VI B,
c) Rhenium und/oder Zinn und
d) mindestens eine Verbindung eines Elements aus den Gruppen I A, II A, III A, III B und/oder von Zink
enthält.

2. Verfahren nach Anspruch 1, **dadurch gekennzeichnet, daß** Propylen aus Propan gewonnen wird.

3. Katalysator insbesondere zur Herstellung von olefinisch ungesättigten Kohlenwasserstoffen aus entsprechenden gesättigten Kohlenwasserstoffen durch Dehydrierung, enthaltend
a) mindestens ein Oxid mindestens eines Elements aus der Gruppe IV B des Periodensystems der Elemente mit mehr als 90 % einer kristallinen Modifikation,
b) mindestens ein Element aus den Gruppen VIII und VIB,
c) Rhenium und/oder Zinn und
d) mindestens eine Verbindung eines Elements aus den Gruppen I A, II A, III A, III B und/oder von Zink.

4. Katalysator nach Anspruch 3, enthaltend Zirkonoxid.

5. Katalysator nach Anspruch 4, enthaltend Zirkonoxid in der tetragonalen Modifikation.

6. Katalysator nach Anspruch 3, enthaltend Titanoxid.

7. Katalysator nach Anspruch 3, enthaltend 0,005 bis 5 Gew.-% Palladium, Platin, Rhodium und/oder Rhenium.

8. Katalysator nach Anspruch 3, enthaltend als Verbindung eines Elements der Gruppe I A eine Natrium- oder Kaliumverbindung.

9. Katalysator nach Anspruch 3, enthaltend als Verbindung eines Elements der Gruppen III A oder III B eine Lanthan-, Yttrium-, Gallium-, Indium- oder Thalliumverbindung.

10. Katalysator nach Anspruch 3, enthaltend als Element der Gruppe VI B Chrom und/oder Wolfram.

11. Katalysator nach einem der Ansprüche 3 bis 10, **gekennzeichnet durch** eine BET-Oberfläche zwischen 10 und 500 m²/g.

12. Katalysator nach einem der Ansprüche 3 bis 11, **gekennzeichnet durch** Poren einer Weite von 2 bis 60 nm, wobei mindestens 10% der Poren eine Weite von mehr als 20 nm aufweisen und das spezifische Porenvolumen 0.1 bis 1 ml/g beträgt.

## Claims

1. A process for preparing olefins having from two to five carbon atoms in the longest chain by dehydrogenation of corresponding paraffinic hydrocarbons over a catalyst, wherein the catalyst comprises
a) at least one oxide of at least one element of group IV B of the Periodic Table of the Elements comprising more than 90% of one crystalline modification,
b) at least one element of groups VIII and VI B,
c) rhenium and/or tin and
d) at least one compound of an element of groups I A, II A, III A, III B and/or of zinc.

2. A process as claimed in claim 1, wherein propylene is obtained from propane.

3. A catalyst, particularly for the preparation of
a) at least one oxide of at least one element of group IV B of the Periodic Table of the Elements comprising more than 90% of one crystalline modification,
b) at least one element of groups VIII and VI B,
c) rhenium and/or tin and
d) at least one compound of an element of groups I A, II A, III A, III B and/or of zinc.

4. A catalyst as claimed in claim 3 comprising zirconium oxide.

5. A catalyst as claimed in claim 4 comprising zirconium oxide in the tetragonal modification.

6. A catalyst as claimed in claim 3 comprising titanium oxide.

7. A catalyst as claimed in claim 3 comprising from 0.005 to 5% by weight of palladium, platinum, rhodium and/or rhenium.

8. A catalyst as claimed in claim 3 comprising a sodium or potassium compound as compound of an element of group I A.

9. A catalyst as claimed in claim 3 comprising a lanthanum, yttrium, gallium, indium or thallium compound as compound of an element of group III A or III B.

10. A catalyst as claimed in claim 3 comprising chromium and/or tungsten as element of group VI B.

11. A catalyst as claimed in any of claims 3 to 10 having a BET surface area of from 10 to 500 m²/g.

12. A catalyst as claimed in any of claims 3 to 11 which has pores having a width of from 2 to 60 nm, where at least 10% of the pores have a width of more than 20 nm and the specific pore volume is from 0.1 to 1 ml/g.

## Revendications

1. Procédé de production d'oléfines présentant, dans leur chaîne la plus longue, deux à cinq atomes de carbone, au moyen d'une déshydrogénation des hydrocarbures paraffiniques correspondantes, sur un catalyseur, **caractérisé en ce que** le catalyseur contient
a) au moins un oxyde d'au moins un élément du groupe IV B de la Classification Périodique des Eléments, présentant une modification cristalline à raison de plus de 90%,
b) au moins un élément des groupes VIII et VI B,
c) du rhénium et/ou de l'étain et
c) au moins un composé d'un élément des groupes I A, II A, III A, III B et/ou de zinc.

2. Procédé selon la revendication 1, **caractérisé en ce que** l'on prépare le propylène à partir du propane.

3. Catalyseur destiné en particulier à la production d'hydrocarbures à insaturation oléfinique, à partir des hydrocarbures saturés correspondants, au moyen d'une déshydrogénation, qui comprend
a) au moins un oxyde d'au moins un élément du groupe IV B de la Classification Périodique des Eléments, présentant une modification cristalline à raison de plus de 90%,
b) au moins un élément des groupes VIII et VI B,
c) du rhénium et/ou de l'étain et
d) au moins un composé d'un élément des groupes I A, II A, III A, III B et/ou de zinc.

4. Catalyseur selon la revendication 3, contenant de l'oxyde de zirconium.

5. Catalyseur selon la revendication 4, contenant de l'oxyde de zirconium dans la modification tétragonale.

6. Catalyseur selon la revendication 3, contenant de l'oxyde de titane.

7. Catalyseur selon la revendication 3, contenant du palladium, du platine, du rhodium et/ou du rhénium, à raison de 0,005% à 5% en poids.

8. Catalyseur selon la revendication 3, contenant, en tant que composé d'un élément du groupe I A, un composé de sodium ou de potassium.

9. Catalyseur selon la revendication 3, contenant, en tant que composé d'un élément du groupe III A ou III B, un composé de lanthane, d'yttrium, de gallium, d'indium ou de thallium.

10. Catalyseur selon la revendication 3, contenant, en tant qu'élément du groupe VI B, du chrome et/ou du tungstène.

11. Catalyseur selon une quelconque des revendications 3 à 10, **caractérisé par** une surface BET comprise entre 10 m²/g et 500 m²/g.

12. Catalyseur selon une quelconque des revendications 3 à 11, **caractérisé par** des pores présentant une largeur comprise entre 2 nm et 60 nm, au moins 10% des pores présentant une largeur supérieure à 20 nm et le volume spécifique des pores étant compris entre 0,1 ml/g et 1 ml/g.
